(19) Europäisches Patentamt
European Patent Office
Office européen des brevets

(11) EP 4 257 157 A1

(12) EUROPEAN PATENT APPLICATION
published in accordance with Art. 153(4) EPC

(43) Date of publication:
**11.10.2023 Bulletin 2023/41**

(21) Application number: **21900784.6**

(22) Date of filing: **05.10.2021**

(51) International Patent Classification (IPC):
***A61L 24/00*** (2006.01) ***A61L 24/04*** (2006.01)
***C08L 71/02*** (2006.01) ***C08K 5/46*** (2006.01)

(52) Cooperative Patent Classification (CPC):
**A61L 24/00; A61L 24/04; C08K 5/46; C08L 71/02;**
Y02A 50/30

(86) International application number:
**PCT/KR2021/013596**

(87) International publication number:
**WO 2022/119096 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2020 KR 20200166202**

(71) Applicant: **Theracion Biomedical Co. Ltd.**
**Jungwon-gu**
**Seongnam-si, Gyeonggi-do 13201 (KR)**

(72) Inventors:
- **KIM, Eun Jin**
**Seongnam-si Gyeonggi-do 13587 (KR)**
- **KIM, Byung Nam**
**Seongnam-si Gyeonggi-do 13228 (KR)**

(74) Representative: **Klunker IP**
**Patentanwälte PartG mbB**
**Destouchesstraße 68**
**80796 München (DE)**

# (54) ABSORBENT BONE HEMOSTATIC MATERIAL COMPOSITION COMPRISING ANTIBIOTIC AND PREPARATION METHOD THEREFOR

(57) The present invention relates to an absorbent bone hemostatic material composition including an antibiotic and a preparation method therefor, and more specifically, to an absorbent bone hemostatic material that provides a physical protective film for stopping bone hemorrhage during surgery, thereby exhibiting an immediate hemostatic effect and preventing infection and inflammatory response at the surgical site.

[FIG. 1]

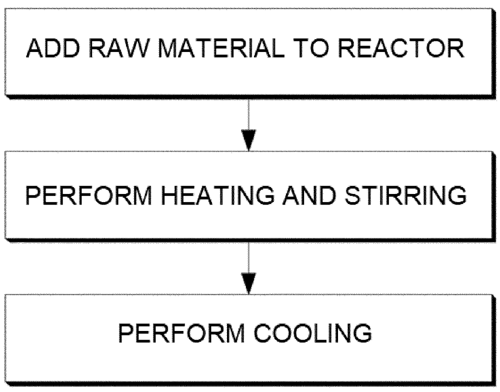


EP 4 257 157 A1

## Description

TECHNICAL FIELD

The present invention relates to an absorbent bone hemostatic material composition including an antibiotic and a preparation method therefor, and more specifically, to an absorbent bone hemostatic material that provides a physical protective film for stopping bone hemorrhage during surgery, thereby exhibiting an immediate hemostatic effect and preventing infection and inflammatory response at the surgical site.

BACKGROUND ART

Bleeding is one of the obstacles that make surgery difficult, and it is very important to stabilize bleeding because a lot of blood loss may affect normal body functions. Hemostasis requires the use of blood coagulants or vascular occlusion devices such as gauze or hemostatic clamps. This type of hemostatic material utilizes the property of slowly activating coagulation factors or narrowing severed blood vessels. Numerous hemostatic materials have already been developed for application of soft tissue bleeding, but there are limitations in that the hemostatic materials cannot be applied to bone bleeding. This is because the blood flow during bone bleeding prevents the stable formation of blood clots, and the bone tissue itself maintains a structure through this condition. In the past, there have been many hemostatic materials that include non-biocompatible materials and thus remain in the body without being completely biodegraded. As a result of many studies, it has been confirmed that the components of the bone hemostatic materials have a negative effect on bone regeneration when the components of the bone hemostatic materials remain unbiodegraded.
Korean Patent Publication No. 1 0-2014-01 07429 discloses a hemostatic agent and a method of using the same. The hemostatic agent includes reverse micelles having an outer hydrophobic shell of suitable biocompatible hydrophobic components such as alkanes, and hydrophilic positively charged chitosan moieties enclosed within the hydrophobic shell, thereby attenuating or stopping the bleeding. However, there are limitations in that the application to bone hemostasis and the immediate hemostasis by a physical protective film are not disclosed.
Korean Patent Publication No. 10-2018-0055747 discloses a novel hemostatic agent including a mussel adhesive protein and a method for preparing an absorbent bone hemostatic agent by using the same. The application to bone hemostasis is not disclosed. Since a lot of auxiliary materials such as nanofiber hemostatic dressings, hemostatic sponges and patches are required and it is difficult to biodegrade well in vivo, it is highly likely to interfere with bone formation. Therefore, there is a need to develop a bone hemostatic material that is quickly absorbed and biodegraded so as not to interfere with bone formation and has an immediate hemostatic effect.
Accordingly, the inventors of the present application have studied to solve the above problems and found that a bone hemostatic material composition including a specific polymer compound and an antibiotic had an immediate hemostatic effect, had a minimal effect on bone regeneration, and could also prevent infection and inflammatory response at the surgical site. In this manner, the inventors of the present application completed the present invention.

DESCRIPTION OF EMBODIMENTS

TECHNICAL PROBLEM

The present invention has been made in an effort to solve the problems of the related art and aims to provide an absorbent bone hemostatic material composition including an antibiotic.
In addition, the present invention aims to provide an absorbent bone hemostatic material including the absorbent bone hemostatic material composition.
In addition, the present invention aims to provide a method for preparing the absorbent bone hemostatic material composition.

SOLUTION TO PROBLEM

As a technical means for achieving the above technical objectives, an aspect of the present invention provides an absorbent bone hemostatic material composition.
The absorbent bone hemostatic material composition includes: a first polymer including a compound represented by Formula 1 or a salt thereof as an active ingredient; a second polymer including a compound represented by Formula 2 or a salt thereof as an active ingredient; and an antibiotic.

[Formula 1]

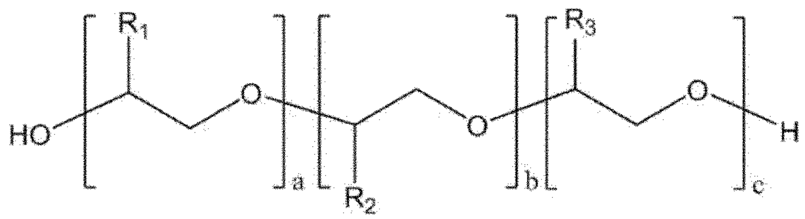

In Formula 1, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c are each independently an integer of 2 to 128, and b is an integer of 16 to 67.

[Formula 2]

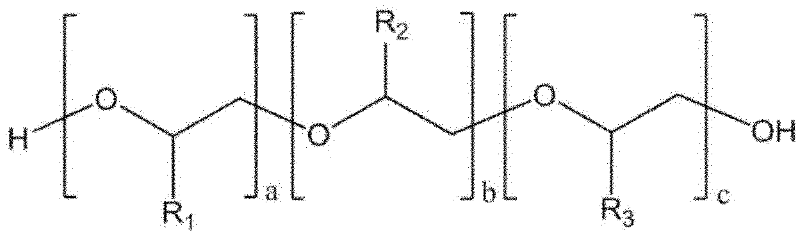

In Formula 2, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c are each independently an integer of 2 to 128, and b is an integer of 16 to 67.

In Formulae 1 and 2, R1 to R3 may each independently be hydrogen, linear or branched C1-C5 alkyl, linear or branched C2-C5 alkenyl, or linear or branched C2-C5 alkynyl.

In Formulae 1 and 2, R1 to R3 may each independently be hydrogen or linear or branched C1-C5 alkyl.

In Formulae 1 to 2, R1 and R3 may each be hydrogen, and R2 may be linear or branched C1-C3 alkyl.

The antibiotic may include a material selected from the group consisting of first-generation cephapirin, second-generation ceftazidime, third-generation cefotaxime, and fourth-generation cefepime in cephalosporins, piperacillin in penicillins, gentamicin, amikacin, tobramycin, and neomycin in aminoglycosides, first-generation norfloxacin, second-generation ciprofloxacin, third-generation levofloxacin, and fourth-generation trovafloxacin in quinolones, tetracycline in tetracyclines, ertapenem, meropenem, and imipenem/cilastatin in carbapenems, vancomycin in glycopeptides, complexes thereof, derivatives thereof, and combinations thereof.

50-70 parts by weight of the second polymer and 0.1-20 parts by weight of the antibiotic may be included based on 30-50 parts by weight of the first polymer.

A weight average molecular weight of the first polymer may be 7,000 to 10,000.

A weight average molecular weight of the second polymer may be 6,000 to 12,000.

The absorbent bone hemostatic material composition may be biodegraded by 90% or more after 3 hours in a phosphate buffer solution (PBS) of pH 7 to 8 at a temperature of 30°C to 37°C.

The absorbent bone hemostatic material composition may exhibit an immediate hemostatic ability of 0-10 seconds.

The absorbent bone hemostatic material composition may have a bacteriostatic reduction rate of 99% or more after 7 days against staphylococcus aureus or escherichia coli.

In addition, another aspect of the present invention provides an absorbent bone hemostatic material.

The absorbent bone hemostatic material includes the absorbent bone hemostatic material composition.

The absorbent bone hemostatic material may be at least one formulation selected from semi-solid formulation and solid formulation.

In addition, another aspect of the present invention provides a method for preparing an absorbent bone hemostatic material composition.

The method for preparing the absorbent bone hemostatic material composition includes: adding, to a reactor, a first polymer including a compound represented by Formula 1 or a salt thereof as an active ingredient, a second polymer including a compound represented by Formula 2 or a salt thereof as an active ingredient, and an antibiotic; heating and stirring the mixture added to the reactor at a temperature of 50°C to 100°C; and cooling the mixture.

[Formula 1]

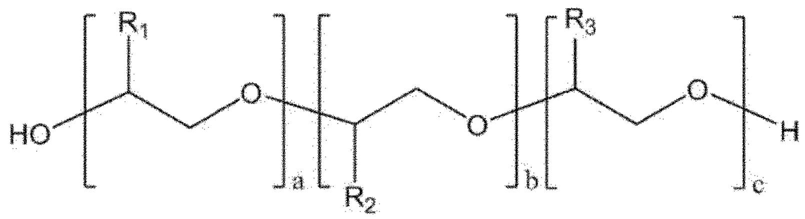

In Formula 1, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c are each independently an integer of 2 to 128, and b is an integer of 16 to 67.

[Formula 2]

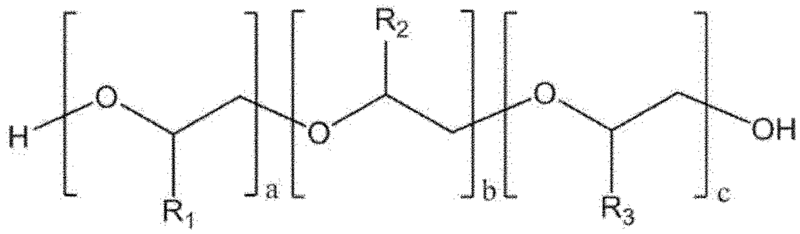

In Formula 2, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c are each independently an integer of 2 to 128, and b is an integer of 16 to 67.

ADVANTAGEOUS EFFECTS OF DISCLOSURE

As described above, the absorbent bone hemostatic material composition according to the present invention provides an effect of being quickly absorbed and biodegraded in vivo.

The absorbent bone hemostatic material composition according to the present invention provides an immediate hemostatic effect.

The absorbent bone hemostatic material composition according to the present invention has an effect of being easily molded into a desired shape because the hardness of the absorbent bone hemostatic material composition varies with temperature. Accordingly, the absorbent bone hemostatic material composition according to the present invention provides an effect of increasing adhesive force.

The absorbent bone hemostatic material composition according to the present invention includes minimal materials, is harmless to the human body, and mixes properly so that the finished product has the effect of the raw material as it is.

The absorbent bone hemostatic material composition according to the present invention provides the effect of capable of preventing infection and inflammatory response at the surgical site.

BRIEF DESCRIPTION OF DRAWINGS

FIG. 1 is a flowchart schematically illustrating a method for preparing an absorbent bone hemostatic material composition according to an embodiment of the present invention.

FIG. 2 is a graph showing the biodegradability of the absorbent bone hemostatic material composition according to an embodiment of the present invention.

FIG. 3 is a graph showing the compressive strength of bone hemostatic material compositions according to Examples of the present invention and Comparative Example.

FIG. 4 is a graph showing the adhesive strength of bone hemostatic material compositions according to Examples of the present invention and Comparative Example.

FIG. 5A to 5C are graphs showing the drug release ability of absorbent bone hemostatic material compositions

according to Examples of the present invention.

FIG. 6 is a graph showing the hemostasis evaluation results of the absorbent bone hemostatic material composition over time according to an embodiment of the present invention.

FIGS. 7A and 7B are photographs showing the biodegradability of the bone hemostatic material composition according to an embodiment of the present invention in in vivo animal tests, respectively.

FIGS. 8A and 8B are graphs showing the antibacterial effect of the bone hemostatic material compositions over time according to Example of the present invention and Comparative Example, respectively, and FIGS. 8C and 8D are photographs showing the antibacterial effect of the bone hemostatic material compositions over time according to Example of the present invention and Comparative Example, respectively.

FIGS. 9A to 9D are photographs showing the inflammatory response and bone regeneration effect of the bone hemostatic material compositions over time according to Examples of the present invention and Comparative Examples.

## BEST MODE FOR CARRYING OUT THE INVENTION

Hereinafter, examples of the present invention will be described so that those of ordinary skill in the art may easily carry out the present invention. However, the present disclosure may be implemented in various different forms and is not limited to the embodiments described herein.

### Example 1. Preparation of absorbent bone hemostatic material composition (including 120 mg/g of antibiotic)

In order to prepare the absorbent bone hemostatic material composition according to the present invention, 34.8 wt% of poloxamer 188, 54.5 wt% of polyethylene glycol-ran-propylene glycol, and 10.7 wt% of gentamicin sulfate were added to a reactor. Thereafter, the mixture introduced into the reactor was melted at a temperature of 80°C and stirred for a sufficient time. Thereafter, the melted and stirred mixture was poured into a mold, placed in a cooler, and cooled at a temperature of 4°C for 30 minutes to prepare a solidified absorbent bone hemostatic material composition (TWG-120).

### Example 2. Preparation of absorbent bone hemostatic material composition (including 60 mg/g of antibiotic)

A solidified absorbent bone hemostatic material composition (TWG-60) was prepared in the same manner as in Example 1, except that 36.8 wt% of poloxamer 188, 57.5 wt% of polyethylene glycol-ran-propylene glycol, and 5.7 wt% of gentamicin sulfate were added to the reactor.

### Example 3. Preparation of absorbent bone hemostatic material composition (including 30 mg/g of antibiotic)

A solidified absorbent bone hemostatic material composition (TWG-30) was prepared in the same manner as in Example 1, except that 37.9 wt% of poloxamer 188, 59.2 wt% of polyethylene glycol-ran-propylene glycol, and 2.9 wt% of gentamicin sulfate were added to the reactor.

### Comparative Example 1. Preparation of absorbent bone hemostatic material composition (including no antibiotic)

A solidified absorbent bone hemostatic material composition (Tableau Wax) was prepared in the same manner as in Example 1, except that 39 wt% of poloxamer 188 and 61 wt% of polyethylene glycol-ran-propylene glycol were added to the reactor and no antibiotic was added thereto.

### Experimental Example 1. Biodegradability measurement experiment (Dissolution test)

In order to measure the biodegradability of the absorbent bone hemostatic material compositions prepared in Examples 1 to 3 over time, 1 g of the absorbent bone hemostatic material compositions prepared in Examples 1 to 3 was precisely weighted in 20 mL of a phosphate buffer solution (PBS, Ph 7.4) at 37°C, and placed in a conical tube, taken out at a set time in a shaking bath (37°C, 60RPM), and the amount of the bone hemostatic material biodegraded and reduced was measured as a weight loss rate. The results of calculating the biodegradation according to Equation 1 are shown in Table 1 and FIG. 2 below.

[Equation 1]

$$Biodegradability = \frac{Initial\ sample\ weight - sample\ weight\ after\ 48\ hours}{Initial\ sample\ weight} \times 100$$

[Table 1]

| Time (h) | Biodegradability (%) | | |
|---|---|---|---|
| | Example 1 | Example 2 | Example 3 |
| 3 | 98.2 | 93.8 | 97.3 |
| 6 | 98.3 | 98.0 | 98.6 |
| 12 | 98.3 | 98.4 | 98.2 |
| 24 | 98.1 | 98.1 | 99.1 |
| 48 | 98.8 | 99.2 | 99.0 |

Referring to Table 1 and FIG. 2, the absorbent bone hemostatic material compositions of Examples 1 to 3 according to the present invention showed a biodegradability of 90% or more after 3 hours and showed a biodegradability of about 98% or more after 6 hours, and it was confirmed that the biodegradability was very excellent. Therefore, even when applied to an actual surgical site, almost all the compositions were biodegraded within 48 hours and it was expected to have a positive effect on bone regeneration.

Experimental Example 2. Mechanical property analysis

The mechanical properties of the absorbent bone hemostatic material compositions prepared in Examples 1 to 3 and Comparative Example 1 were analyzed. First, analog force gauge (HANDPI NK-10) was used to analyze the compressive strength. Specifically, a sample was placed on a flat plane and a compression force measuring jig of the measuring equipment was mounted at the upper end. Thereafter, the maximum force up to the point at which the sample broke was measured and recorded, and the results are shown in FIG. 3. As illustrated in FIG. 3, it could be confirmed that the hemostatic material composition of Example 1 showed relatively high compressive strength, but the hemostatic material compositions of Examples 2 and 3 and Comparative Example 1 showed almost similar compressive strength.

Meanwhile, in order to analyze the adhesive strength, 0.1 g of a sample was kneaded to form a sphere with a diameter of 3 mm and then pressed to a thickness of 1 mm by using the circular jig of the measuring equipment. Thereafter, while lifting the measuring equipment upward, the adhesive strength of the sample was measured and recorded, and the results thereof are shown in FIG. 4. As illustrated in FIG. 4, it could be confirmed that the hemostatic material composition of Example 1 showed relatively low adhesive strength, the hemostatic material composition of Comparative Example 1 showed relatively high adhesive strength, and the hemostatic material compositions of Examples 2 and 3 showed almost similar adhesive strength.

Therefore, it could be confirmed that the hemostatic material compositions of Examples 1 to 3 and Comparative Example 1 did not significantly differ in their physical properties and had almost similar mechanical properties.

Experimental Example 3. Drug release activity measurement experiment

The drug release abilities of the absorbent bone hemostatic material compositions prepared in Examples 1 to 3 were measured. Since gentamicin sulfate added as an antibiotic did not have a wavelength detectable by a UV detector, the measurement was performed by using HPLC.

Specifically, after drawing a calibration curve of standard samples 200, 250, 500 and 1,000 ppm, the concentrations of the respective samples were calculated by using a linear regression equation. At this time, the conditions of the test were as follows:

(1) Column: 4.6 x 150 mm, 5 μm, C18
(2) Mobile Phase: 0.2% TFA: MeOH (98:2)
(3) flow rate: 1 mL/min
(4) Analysis time: 20 min
(5) ELSD Temperature: 60°C

(6) ELSD gain: 7
(7) Temperature: 30°C
(8) Injection volume: 10 μL

Thereafter, the drug release ability over time was measured and shown in FIGS. 5A to 5C. Referring to FIGS. 5A to 5C, all the absorbent bone hemostatic material compositions prepared in Examples 1 to 3 showed the drug release ability of 100% at 2 hours. This is a result showing a trend similar to the biodegradability measurement experiment. It could be expected that the absorbent bone hemostatic material composition prepared according to the present invention was efficiently applicable to a local site because biodegradation occurred and the drug was immediately released.

Experimental Example 4. Sterility and antibacterial evaluation test

In order to evaluate the sterility of the absorbent bone hemostatic material compositions prepared in Examples 1 to 3, a test was conducted according to the sterility test method, the eleventh edition of the Korean Pharmacopoeia. The sterility test is a test widely used as a means for evaluating the suitability of sterilization for various medical devices and test substances, and this test evaluated the sterility of the test substance by directly immersing the test substance in the medium.

First, a portion of each of the absorbent bone hemostatic material compositions prepared in Examples 1 to 3 was inoculated into a liquid thioglycolic acid medium and a soybean casein digestion medium. At this time, the liquid thioglycolic acid medium was cultured at 30°C to 35°C, and the soybean casein digestion medium was cultured at 20°C to 25°C. On the seventh and fourteenth days after culture, the presence or absence of proliferation of bacteria was observed and shown in Table 2 below.

[Table 2]

| Medium | Classification | Interim inspection date (7 days) | Last inspection date (14 days) | Determination |
|---|---|---|---|---|
| Liquid thioglycolic acid medium | Test material | N.G. | N.G. | Suitable |
| | Negative control | N.G. | N.G. | |
| Soybean casein digestion medium | Test material | N.G. | N.G. | Suitable |
| | Negative control | N.G. | N.G. | |

As illustrated in Table 2, as a result of the sterility test of the absorbent bone hemostatic material composition according to the present invention, the proliferation of bacteria could not be confirmed in both the liquid thioglycolic acid medium and the soybean casein digestion medium. Therefore, the absorbent bone hemostatic material composition was determined to be suitable for the eleventh edition of the Korean Pharmacopoeia, the test standard for the sterility test method.

In addition, the antibacterial test was conducted to measure the effect of antibiotic injection. Specifically, staphylococcus aureus and escherichia coli were applied to the experiment, and the number of bacteria was measured after 24 hours to measure the antibacterial activity. As a result of the test, both staphylococcus aureus and escherichia coli showed an antibacterial activity value of 99.9% or more, and it was confirmed that the antibiotic applied to the absorbent bone hemostatic material composition according to the present invention effectively exhibited its effect.

Experimental Example 5. In vivo efficacy evaluation

In order to evaluate the in vivo efficacy of the absorbent bone hemostatic material compositions prepared in Example 3 and Comparative Example 1, bone infection was induced in a rat skull defect model by using S. aureus, and each hemostatic material composition was applied thereon. Then, antibiotic effect and safety were evaluated. In addition, the results were compared through the biopsy and the evaluation of biodegradability, hemostasis ability, and inflammatory properties in the blood after transplantation. Meanwhile, groups of rats used in each experiment are summarized and shown in Table 3 below.

[Table 3]

| Bacterial count change, inflammation level, histology evaluation | | | |
|---|---|---|---|
| Group 1 (day 3) | | | |
| Group 1-1 (infection X, Example 3) | Group 1-2 (infection O, Example 3) | Group 1-3 (infection X, Comparative Example 1) | Group 1-4 (infection O, Comparative Example 1) |
| Group 2 (week 1) | | | |
| Group 2-1 (infection X, Example 3) | Group 2-2 (infection O, Example 3) | Group 2-3 (infection X, Comparative Example 1) | Group 2-4 (infection O, Comparative Example 1) |
| Group 3 (week 2) | | | |
| Group 3-1 | Group 3-2 | Group 3-3 | Group 3-4 |
| (infection X, Example 3) | (infection O, Example 3) | (infection X, Comparative Example 1) | (infection O, Comparative Example 1) |
| Group 4 (week 4) | | | |
| Group 4-1 (infection X, Example 3) | Group 4-2 (infection O, Example 3) | Group 4-3 (infection X, Comparative Example 1) | Group 4-4 (infection O, Comparative Example 1) |
| 5 rats | 5 rats | 5 rats | 5 rats |
| Immediate hemostasis ability, biodegradability evaluation | | | |
| Group 5 | | | |
| Group 5-1 (day 3) | | Group 5-2 (week 1) | |
| 10 rats | | 10 rats | |

(1) Immediate hemostasis ability evaluation

In order to evaluate the immediate hemostasis ability of the absorbent bone hemostatic material compositions prepared in Example 3 and Comparative Example 1, the absorbent bone hemostatic material compositions were applied on each wound, and the time point of hemostasis from 0 seconds to 10 seconds was evaluated and recorded in seconds through video recording. The time point was divided into ① 0-1 second, ② 2-4 seconds, ③ 5-7 seconds, ④ 8-9 seconds, and ⑤ more than 10 seconds and the evaluation was performed. Meanwhile, the results of the immediate hemostasis evaluation of Example 3 are shown in FIG. 6.

As illustrated in FIG. 6, the absorbent bone hemostatic material composition prepared according to Example 3 of the present invention was evaluated for a total of 40 defects, each two skull defects in 20 subjects, and it was confirmed that the absorbent bone hemostatic material composition showed immediate hemostasis ability of 85%. Meanwhile, it was confirmed that the absorbent bone hemostatic material composition prepared according to Comparative Example 1 showed an immediate hemostasis ability of 73%, and the absorbent bone hemostatic material composition according to the present invention had more excellent immediate hemostasis ability than that of Comparative Example.

(2) Biodegradability evaluation

In order to evaluate the biodegradability of the absorbent bone hemostatic material composition prepared in Example 3, autopsies were performed 3 days and 7 days (1 week) after the immediate hemostasis evaluation of the skull of the rat to confirm whether the formulation remained around the wound. The results are shown in FIG. 7A (3 days) and FIG. 7B (7 days). As a result of visual evaluation, it could be confirmed that the formulation was completely biodegraded and disappeared at 3 days and 1 week, and thus, it could be confirmed that the absorbent bone hemostatic material composition according to the present invention had excellent biodegradability.

(3) Antibacterial evaluation

In order to evaluate the antibacterial properties of the absorbent bone hemostatic material compositions prepared in Example 3 and Comparative Example 1, groups were classified into groups 1-1 and 2-1 (SXGO), groups 1-2

and 2-2 (SOGO), groups 1-3 and 2-3 (SXGX), and groups 1-4 and 2-4 (SOGX), and antibacterial activity was evaluated through the presence or absence of bacterial growth in each group. At this time, as shown in Table 3, SO was an S. aureus infected group, SX was an S. aureus non-infected group, GO was a gentamicin-containing group, and GX was a gentamicin-free group. First, after implanting a gelatin sponge on a rat skull, $10^7$ CFU bacteria were dropped on both sides, and the number of bacteria was measured after swabbing the gelatin sponge on both sides with a cotton swab on each observation day. In addition, the results thereof are shown in FIGS. 8A to 8D.

First, FIGS. 8A and 8B are graphs showing the number of bacteria (CFU) for each group after 3 days and 1 week, respectively. As shown in the above graph, it was confirmed that the number of bacteria in the group classified as the SOGX group was significantly higher, and the number of bacteria in the group classified as the SOGO group was almost zero. Meanwhile, the photographs on the left sides of FIGS. 8C and 8D show the bacteria of the SOGX group after 3 days and 1 week, respectively, and the photographs on the right sides of FIGS. 8C and 8D show the bacteria of the SOGO group after 3 days and 1 week, respectively. Even with reference to this, it was confirmed that the number of bacteria in the SOGX medium was significantly higher than the number of bacteria in the SOGO medium.

That is, it could be confirmed that the bone hemostatic material composition (SOGO group) prepared according to Example 3 of the present invention had a significantly superior antibacterial effect, compared to the bone hemostatic material composition (SOGX group) prepared according to Comparative Example 1.

(4) Evaluation of inflammatory response and bone regeneration effect through tissue staining

In order to evaluate the inflammatory response and bone regeneration effect of the absorbent bone hemostatic material compositions prepared in Example 3 and Comparative Example 1, the groups were classified into the four groups (SXGO, SOGO, SXGX, and SOGX) as in the section (3) above, and the degree of inflammation and bone formation were confirmed through H&E staining. The results thereof are shown in FIGS. 9A to 9D (FIG. 9A: the third day, FIG. 9B: the first week, FIG. 9C: the second week, FIG. 9D: the fourth week). At this time, as shown in Table 3, SO was an S. aureus infected group, SX was an S. aureus non-infected group, GO was a gentamicin-containing group, and GX was a gentamicin-free group.

Among them, as illustrated in FIG. 9A, it could be confirmed that, in the bone defect part on the third day, the SOGO group had a decrease in inflammatory cells, compared to the SOGX group, and had an increase in the amount and activity of Ostoblast, compared to the other treatment groups.

Accordingly, it could be confirmed that, when applied to the infected bone, the bone hemostatic material composition (SOGO group) prepared according to Example 3 of the present invention showed an excellent effect on inflammation reduction and bone regeneration, compared to the bone hemostatic material composition (SOGX group) prepared according to Comparative Example 1.

MODE OF DISCLOSURE

Hereinafter, the present invention will be described in more detail. However, the present invention may be embodied in various different forms and is not limited by embodiments described herein, and the present invention is only defined by the claims to be described below.

In addition, the terms as used herein are only used to describe specific embodiments, and are not intended to limit the present invention. The singular forms as used herein are intended to include the plural forms as well unless the context clearly indicates otherwise. In the specification of the present invention, the phrase "including a certain element" means "further including other elements" rather than excluding other elements unless otherwise stated.

A first aspect of the present application provides an absorbent bone hemostatic material composition.

The absorbent bone hemostatic material composition includes: a first polymer including a compound represented by Formula 1 or a salt thereof as an active ingredient; a second polymer including a compound represented by Formula 2 or a salt thereof as an active ingredient; and an antibiotic.

[Formula 1]

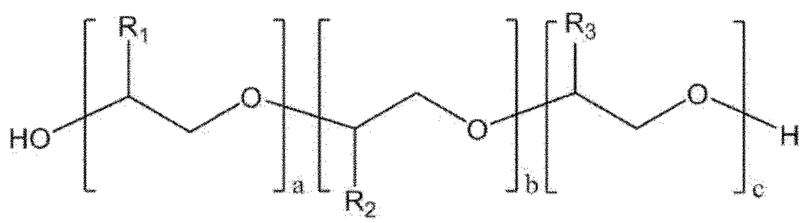

In Formula 1, R1 to R3 may each independently be hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c may each independently be an integer of 2 to 128, and b may be an integer of 16 to 67.

[Formula 2]

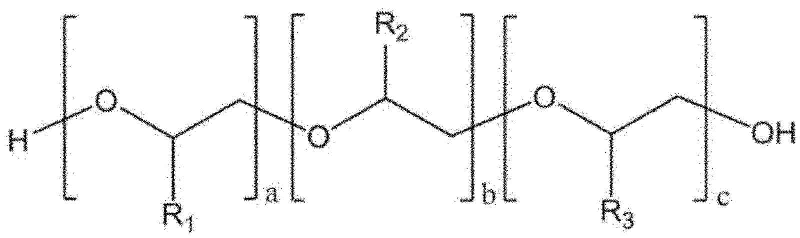

In Formula 2, R1 to R3 may each independently be hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c may each independently be an integer of 2 to 128, and b may be an integer of 16 to 67.

A second aspect of the present application provides an absorbent bone hemostatic material.

The absorbent bone hemostatic material includes the absorbent bone hemostatic material composition according to the first aspect of the present application.

Hereinafter, the absorbent bone hemostatic material composition and the absorbent bone hemostatic material including the same according to the first and second aspects of the present application will be described in detail.

Prior to describing the absorbent bone hemostatic material composition according to the present invention, there has been a problem in that the hemostatic effect of the raw material could not be exhibited 100% in the finished product because the physical properties of the raw material and the physical properties of the finished product were different from each other due to the reaction of each material during mixing while including various materials. In addition, in many cases, a lot of unnecessary materials were included to increase hemostasis ability, and non-compatible materials were included. Therefore, there was also a problem of interfering with bone regeneration. In particular, 'BONE WAX' of Ethicon/J&J is applied to a cut surface to provide a physical protective film, but is non-absorbent and remains on the wound continuously. There was a problem of hindering the induction and growth of normal bone cells. Meanwhile, the absorbent bone hemostatic material composition according to the present invention includes a minimum of highly biocompatible bioabsorbent composition, is almost harmless to the human body, and is quickly absorbed and biodegraded. When applied to the bone surface during fractures and bone cutting, the absorbent bone hemostatic material composition provides a physical protective film and has an immediate hemostatic effect. Therefore, the absorbent bone hemostatic material composition may have an effect that does not interfere with normal bone regeneration. In addition, by further including an antibiotic, the absorbent bone hemostatic material composition may have an effect of preventing infection and inflammatory response at the treatment site.

In an embodiment of the present application, in Formulae 1 and 2, R1 to R3 may each independently be hydrogen, linear or branched C1-C5 alkyl, linear or branched C2-C5 alkenyl, or linear or branched C2-C5 alkynyl. Preferably, in Formulae 1 and 2, R1 to R3 may each independently be hydrogen, linear or branched-chain C1-C5 alkyl. More preferably, in Formulae 1 and 2, R1 and R3 may each be hydrogen, and R2 may be linear or branched-chain C1-C3 alkyl. According to an embodiment of the present invention, the first polymer including the compound represented by Formula 1 or a salt thereof as an active ingredient may be poloxamer, and the second polymer including the compound represented by Formula 2 or a salt thereof as an active ingredient may be polyethylene glycol-ran-propylene glycol. Meanwhile, the poloxamer may have a structure in which polyoxypropylene, which is a hydrophobic repeating unit, is included in a central core, and polyoxyethylene, which is a hydrophilic repeating unit, is formed on both sides.

In an embodiment of the present application, the antibiotic is added to prevent infection and inflammatory response at the treatment site, and may include a material selected from the group consisting of, for example, first-generation cephapirin, second-generation ceftazidime, third-generation cefotaxime, and fourth-generation cefepime in cephalosporins, piperacillin in penicillins, gentamicin, amikacin, tobramycin, and neomycin in aminoglycosides, first-generation norfloxacin, second-generation ciprofloxacin, third-generation levofloxacin, and fourth-generation trovafloxacin in quinolones, tetracycline in tetracyclines, ertapenem, meropenem, and imipenem/cilastatin in carbapenems, vancomycin in glycopeptides, complexes thereof, derivatives thereof, and combinations thereof. According to an embodiment of the present invention, the antibiotic may preferably include gentamicin.

In an embodiment of the present application, 50-70 parts by weight of the second polymer and 0.1-20 parts by weight of the antibiotic may be included based on 30-50 parts by weight of the first polymer. Preferably, 1-20 parts by weight of the antibiotic may be included. When the first polymer and the second polymer are included in excess of the

above range, the mixing of the composition may not be performed properly. As a result, the hemostatic effect may be inhibited or satisfactory absorption or biodegradation performance may be difficult to obtain. In addition, when the antibiotic is included in an amount below the above range, a relatively low content of the antibiotic may cause infection and inflammatory response at the treatment site. When the antibiotic is included in excess of the above range, the content of the first polymer and the content of the second polymer are relatively low, and thus, biodegradability and hemostatic effect may be inhibited. Meanwhile, the first polymer, the second polymer, and the antibiotic may be properly mixed. When the first polymer, the second polymer, and the antibiotic are properly mixed, it may be meaningful in that the characteristics of the raw material may be maintained.

In an embodiment of the present application, the first polymer may have a melting point of 40°C to 100°C. When the melting point is lower than 40°C, there is a problem that it is difficult to obtain a hemostatic effect because it is liquefied at body temperature when using a bone hemostatic material. When the temperature is higher than 100°C, the molecular arrangement of the first polymer and the second polymer may not be properly performed because the melting process has to be performed with high heat during the manufacturing process. Therefore, there may be a problem in that it may be difficult to obtain the absorbent bone hemostatic material composition having immediate hemostasis performance and quick absorption and biodegradation performance of the present invention.

In an embodiment of the present application, a weight average molecular weight of the first polymer may be 7,000 to 10,000, and a weight average molecular weight of the second polymer may be 6,000 to 12,000. When the weight average molecular weight of the first polymer is less than 7,000 or greater than 10,000, or when the weight average molecular weight of the second polymer is less than 6,000 or greater than 12,000, the absorbent bone hemostatic material composition including the same may not have an effect of immediate hemostasis and quick absorption and biodegradation. Meanwhile, the weight average molecular weight of the second polymer may be preferably 6,000 to 9,800.

In an embodiment of the present application, the absorbent bone hemostatic material composition may have a hardness of 40 to 80 HS at a temperature of 20°C to 40°C, and preferably 60 to 80 HS at a temperature of 20°C to 30°C and 40 to 60 HS at a temperature of 30°C to 40°C. When the hardness is less than 40 HS at a temperature of 20°C to 40°C, it may be too soft and may not properly adhere to the bone, and thus, it may be difficult to obtain satisfactory immediate hemostatic performance. In addition, when the hardness is greater than 80 HS at a temperature of 20°C to 40°C, it is too hard to form a desired shape, and thus, it may not properly adhere to the bone and it may be difficult to obtain a satisfactory immediate hemostatic effect.

In an embodiment of the present application, the absorbent bone hemostatic material composition may be biodegraded by 90% or more after 3 hours in a phosphate buffer solution (PBS) of pH 7 to 8 at a temperature of 30°C to 37°C, and preferably 93% or more. In addition, biodegradation may proceed 98% or more after 6 hours under the same conditions, and may proceed about 99% or more after 48 hours. Meanwhile, when biodegradation proceeds for more than 50 hours under the same conditions, an immune response to foreign substances may occur by an immune system, which may interfere with bone formation.

In an embodiment of the present application, the absorbent bone hemostatic material composition may exhibit an immediate hemostatic ability of 0-10 seconds. Specifically, the absorbent bone hemostatic material composition may exhibit excellent immediate hemostatic ability of about 85% immediate hemostasis for 0-1 second, and may complete 100% hemostasis in less than 10 seconds.

In an embodiment of the present application, the absorbent bone hemostatic material composition may have a bacteriostatic reduction rate of 99% or more, preferably about 99.9% or more, after 7 days against staphylococcus aureus or escherichia coli. This may be because the absorbent bone hemostatic material composition includes the antibiotic, and due to this, the absorbent bone hemostatic material composition may have a very excellent antibacterial effect.

In an embodiment of the present application, the absorbent bone hemostatic material composition may exhibit a drug release ability of about 80% or more at 1 hour and may exhibit a drug release ability of about 100% at 2 hours. Therefore, the absorbent bone hemostatic material composition may have an excellent effect of efficiently applying the drug to a local site because the drug is immediately released at the same time as biodegradation.

In an embodiment of the present application, the bone mineral density (BMD) after using the absorbent bone hemostatic material composition may be 0.5 to 0.6, and preferably 0.51.

In an embodiment of the present application, the absorbent bone hemostatic material includes the absorbent bone hemostatic material composition and may be characterized in that it is at least one formulation selected from semi-solid formulation and solid formulation. In addition, the absorbent bone hemostatic material may include a groove in the central portion. As described above, since the absorbent bone hemostatic material includes grooves formed in a row in the central portion, the absorbent bone hemostatic material may have an advantage of being easily detached.

A third aspect of the present application provides a method for preparing an absorbent bone hemostatic material composition.

The method for preparing the absorbent bone hemostatic material composition includes: adding, to a reactor, a first polymer including a compound represented by Formula 1 or a salt thereof as an active ingredient, a second

polymer including a compound represented by Formula 2 or a salt thereof as an active ingredient, and an antibiotic; heating and stirring the mixture added to the reactor at a temperature of 50°C to 100°C; and cooling the mixture.

[Formula 1]

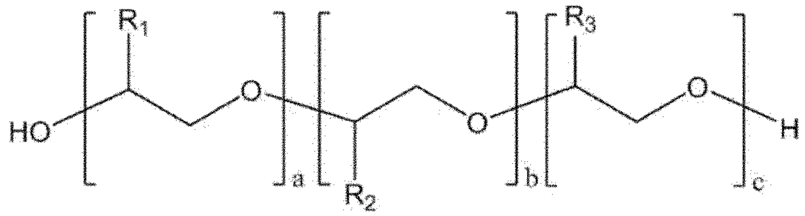

In Formula 1, R1 to R3 may each independently be hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c may each independently be an integer of 2 to 128, and b may be an integer of 16 to 67.

[Formula 2]

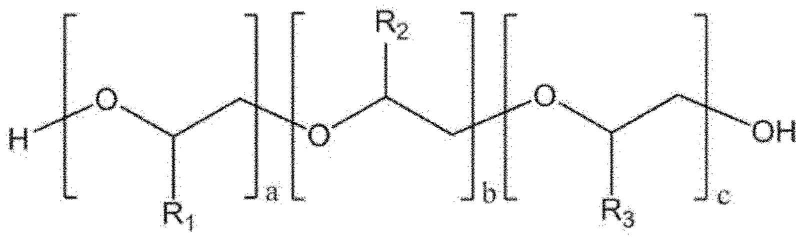

In Formula 2, R1 to R3 may each independently be hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl, a and c may each independently be an integer of 2 to 128, and b may be an integer of 16 to 67.

Although detailed descriptions of parts overlapping the first aspect and the second aspect of the present application are omitted, the descriptions of the first aspect and the second aspect of the present application may be equally applied even when the descriptions thereof are omitted in the third aspect.

Hereinafter, the method for preparing the absorbent bone hemostatic material composition according to the third aspect of the present application will be described in detail with reference to FIG. 1. At this time, FIG. 1 is a flow chart schematically showing the method for preparing the absorbent bone hemostatic material composition.

First, in an embodiment of the present application, the method for preparing the absorbent bone hemostatic material composition may include adding, to a reactor, a first polymer including a compound represented by Formula 1 or a salt thereof as an active ingredient, a second polymer including a compound represented by Formula 2 or a salt thereof as an active ingredient, and an antibiotic.

In an embodiment of the present application, 50-70 parts by weight of the second polymer and 0.1-20 parts by weight of the antibiotic may be added based on 30-50 parts by weight of the first polymer. Preferably, 1-20 parts by weight of the antibiotic may be added. When the first polymer and the second polymer are added in excess of the above range, the mixing of the composition may not be performed properly. As a result, the hemostatic effect may be inhibited or satisfactory absorption or biodegradation performance may be difficult to obtain. In addition, when the antibiotic is added in an amount below the above range, a relatively low content of the antibiotic may cause infection and inflammatory response at the treatment site. When the antibiotic is added in excess of the above range, the content of the first polymer and the content of the second polymer are relatively low, and thus, biodegradability and hemostatic effect may be inhibited. Meanwhile, the first polymer, the second polymer, and the antibiotic may be properly mixed. When the first polymer, the second polymer, and the antibiotic are properly mixed, it may be meaningful in that the characteristics of the raw material may be maintained.

Next, in an embodiment of the present application, the method for preparing the absorbent bone hemostatic material composition may include heating and stirring the mixture added to the reactor at a temperature of 50°C to 100°C.

In an embodiment of the present application, the heating temperature may be preferably about 80°C, and each added material may be melted in the above temperature range. When the heating temperature is lower than 50°C, the first polymer, the second polymer, and the antibiotic may not be smoothly stirred. Accordingly, it may be difficult to obtain an absorbent bone hemostatic material composition having an immediate hemostatic effect. In addition, when the heating

temperature is higher than 100°C, the molecular arrangement of the first polymer and the second polymer may not be properly formed. Accordingly, it may be difficult to obtain an absorbent bone hemostatic material composition having immediate hemostatic performance and quick absorption and biodegradation performance.

Next, in an embodiment of the present application, the method for preparing the absorbent bone hemostatic material composition may include cooling the mixture.

In an embodiment of the present application, the cooling may be performed at a temperature of -30°C to 10°C for 10-60 minutes, and preferably at a temperature of 4°C for 30 minutes. When the cooling is performed out of the above temperature range, it may be difficult to obtain the absorbent bone hemostatic material composition according to the present invention because the molecules are not properly arranged. In addition, even when the cooling is performed for less than 10 minutes, it may be difficult to obtain the absorbent bone hemostatic material composition having satisfactory effects of immediate hemostasis and quick absorption and biodegradation according to the present invention.

Meanwhile, the cooling may be performed by pouring the mixture into a mold and then adding the mixture to a cooler. In this manner, the solidified absorbent bone hemostatic material composition may be prepared.

The present invention has been described in detail with reference to the preferred embodiments and the drawings, but the scope of the technical idea of the present invention is not limited to these drawings and embodiments. Accordingly, various modifications or equivalents thereof may fall within the scope of the technical idea of the present invention. Therefore, the scope of the technical idea according to the present invention should be interpreted by the claims, and the technical idea within the equivalents should be interpreted as falling within the scope of the present invention.

INDUSTRIAL APPLICABILITY

As described above, the absorbent bone hemostatic material composition according to the present invention provides an effect of being quickly absorbed and biodegraded in vivo.

The absorbent bone hemostatic material composition according to the present invention provides an immediate hemostatic effect.

The absorbent bone hemostatic material composition according to the present invention has an effect of being easily molded into a desired shape because the hardness of the absorbent bone hemostatic material composition varies with temperature. Accordingly, the absorbent bone hemostatic material composition according to the present invention provides an effect of increasing adhesive force.

The absorbent bone hemostatic material composition according to the present invention includes minimal materials, is harmless to the human body, and mixes properly so that the finished product has the effect of the raw material as it is.

The absorbent bone hemostatic material composition according to the present invention provides the effect of capable of preventing infection and inflammatory response at the surgical site.

**Claims**

1. An absorbent bone hemostatic material composition comprising:

a first polymer including a compound represented by Formula 1 or a salt thereof as an active ingredient;
a second polymer including a compound represented by Formula 2 or a salt thereof as an active ingredient; and an antibiotic,

[Formula 1]

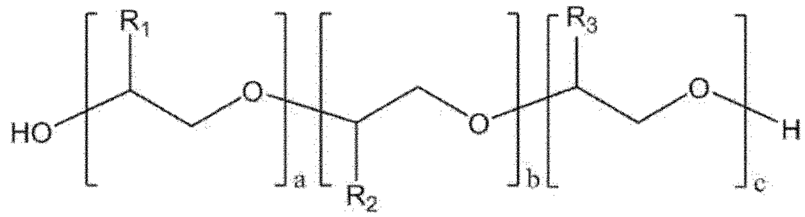

(in Formula 1, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl,

a and c are each independently an integer of 2 to 128, and
b is an integer of 16 to 67.)

[Formula 2]

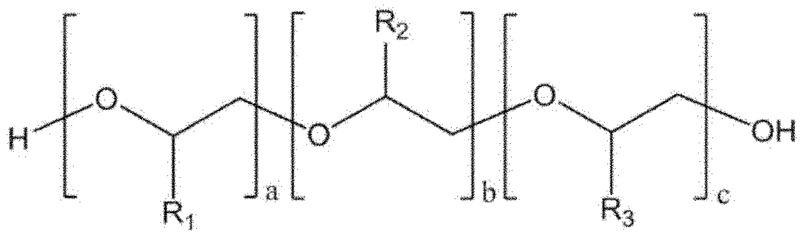

(in Formula 2, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl,
a and c are each independently an integer of 2 to 128, and
b is an integer of 16 to 67.)

2. The absorbent bone hemostatic material composition of claim 1, wherein, in Formulae 1 and 2,
R1 to R3 are each independently hydrogen, linear or branched C1-C5 alkyl, linear or branched C2-C5 alkenyl, or linear or branched C2-C5 alkynyl.

3. The absorbent bone hemostatic material composition of claim 1, wherein,

in Formulae 1 and 2,
R1 to R3 are each independently hydrogen or linear or branched C1-C5 alkyl.

4. The absorbent bone hemostatic material composition of claim 1, wherein,
in Formulae 1 to 2,

R1 and R3 are each hydrogen, and
R2 is linear or branched C1-C3 alkyl.

5. The absorbent bone hemostatic material composition of claim 1, wherein the antibiotic includes a material selected from the group consisting of first-generation cephapirin, second-generation ceftazidime, third-generation cefotaxime, and fourth-generation cefepime in cephalosporins, piperacillin in penicillins, gentamicin, amikacin, tobramycin, and neomycin in aminoglycosides, first-generation norfloxacin, second-generation ciprofloxacin, third-generation levofloxacin, and fourth-generation trovafloxacin in quinolones, tetracycline in tetracyclines, ertapenem, meropenem, and imipenem/cilastatin in carbapenems, vancomycin in glycopeptides, complexes thereof, derivatives thereof, and combinations thereof.

6. The absorbent bone hemostatic material composition of claim 1, wherein 50-70 parts by weight of the second polymer and 0.1-20 parts by weight of the antibiotic are included based on 30-50 parts by weight of the first polymer.

7. The absorbent bone hemostatic material composition of claim 1, wherein a weight average molecular weight of the first polymer is 7,000 to 10,000, and a weight average molecular weight of the second polymer is 6,000 to 12,000.

8. The absorbent bone hemostatic material composition of claim 1, wherein the absorbent bone hemostatic material composition is biodegraded by 90% or more after 3 hours in a phosphate buffer solution (PBS) of pH 7 to 8 at a temperature of 30°C to 37°C.

9. The absorbent bone hemostatic material composition of claim 1, wherein the absorbent bone hemostatic material composition exhibits an immediate hemostatic ability of 0-10 seconds.

10. The absorbent bone hemostatic material composition of claim 1, wherein the absorbent bone hemostatic material composition has a bacteriostatic reduction rate of 99% or more after 7 days against staphylococcus aureus or

escherichia coli.

**11.** An absorbent bone hemostatic material comprising the absorbent bone hemostatic material composition of claim 1.

**12.** The absorbent bone hemostatic material of claim 11, wherein the absorbent bone hemostatic material is at least one formulation selected from semi-solid formulation and solid formulation.

**13.** A method for preparing an absorbent bone hemostatic material composition, the method comprising:

adding, to a reactor, a first polymer including a compound represented by Formula 1 or a salt thereof as an active ingredient, a second polymer including a compound represented by Formula 2 or a salt thereof as an active ingredient, and an antibiotic;
heating and stirring a mixture added to the reactor at a temperature of 50°C to 100°C; and
cooling the mixture,

[Formula 1]

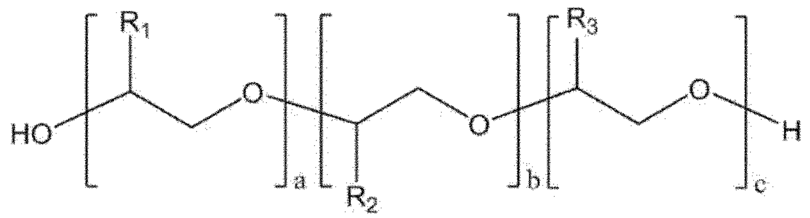

(in Formula 1, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl,
a and c are each independently an integer of 2 to 128, and
b is an integer of 16 to 67.)

[Formula 2]

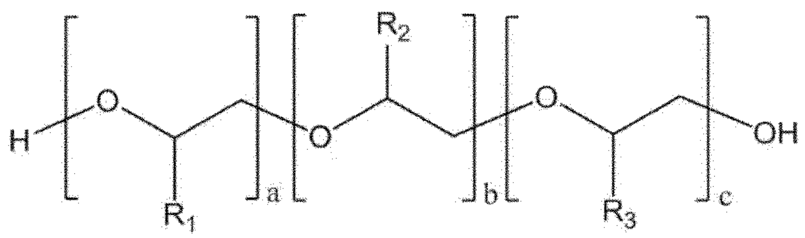

(in Formula 2, R1 to R3 are each independently hydrogen, linear or branched C1-C10 alkyl, linear or branched C1-C10 alkoxy, linear or branched C1-C10 aminoalkyl, linear or branched C2-C10 alkenyl, or linear or branched C2-C10 alkynyl,
a and c are each independently an integer of 2 to 128, and
b is an integer of 16 to 67.)

[FIG. 1]

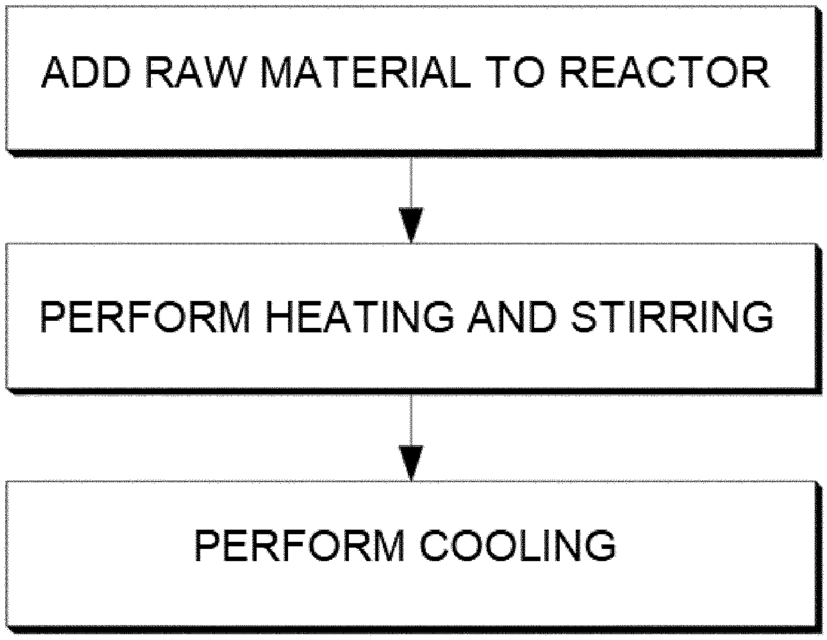
ADD RAW MATERIAL TO REACTOR
PERFORM HEATING AND STIRRING
PERFORM COOLING

FIG. 2

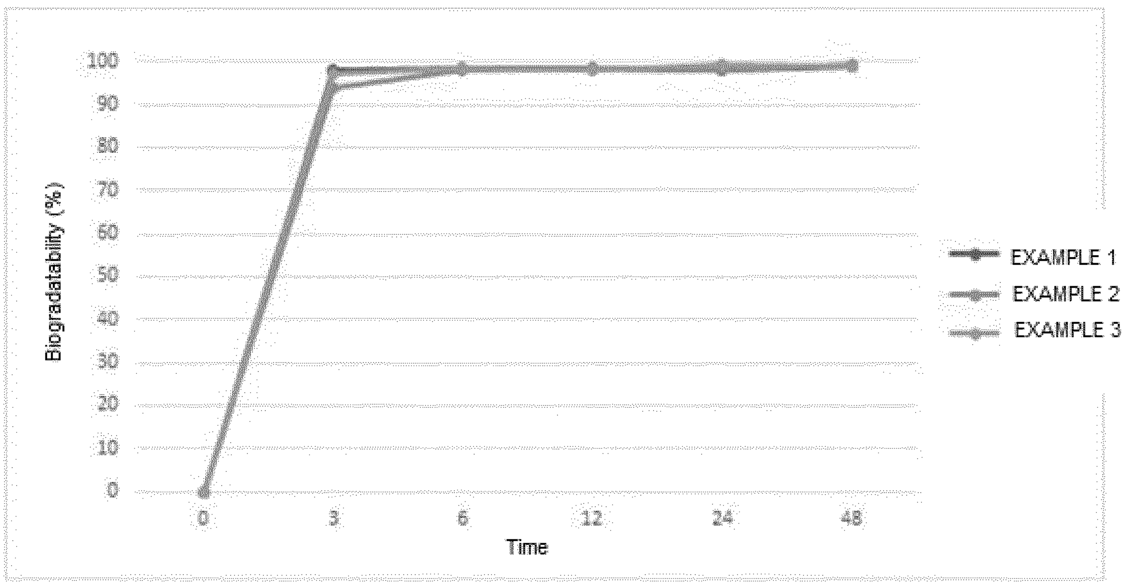
Biogradatability (%)
100
90
80
70
60
50
40
30
20
10
0
0
3
6
12
24
48
Time
EXAMPLE 1
EXAMPLE 2
EXAMPLE 3

FIG. 3

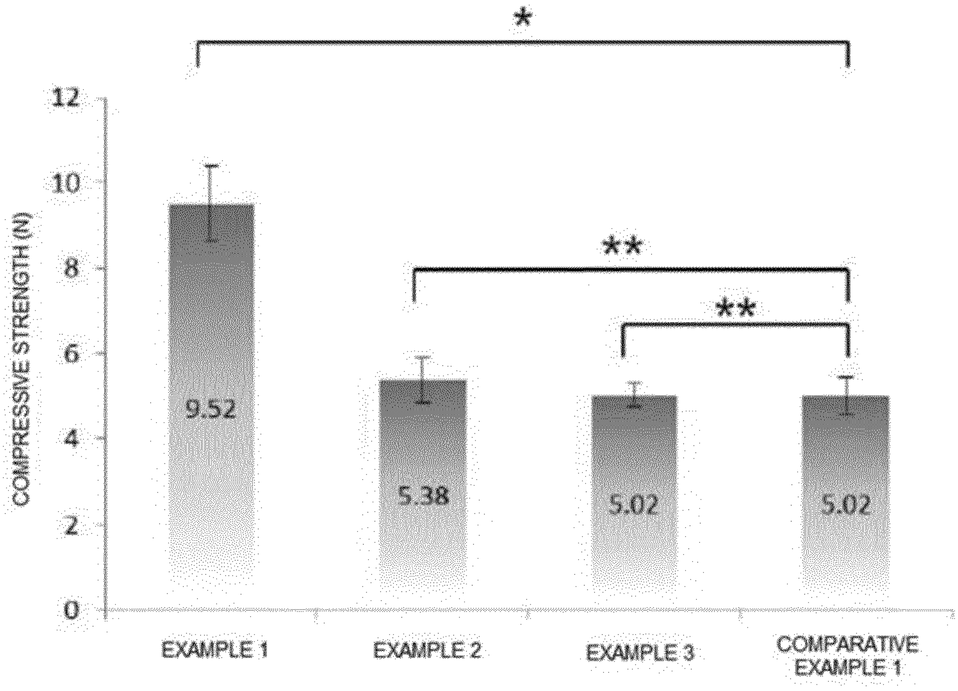
*
**
**
12
10
8
6
4
2
0
COMPRESSIVE STRENGTH (N)
9.52
5.38
5.02
5.02
EXAMPLE 1
EXAMPLE 2
EXAMPLE 3
COMPARATIVE EXAMPLE 1

FIG. 4

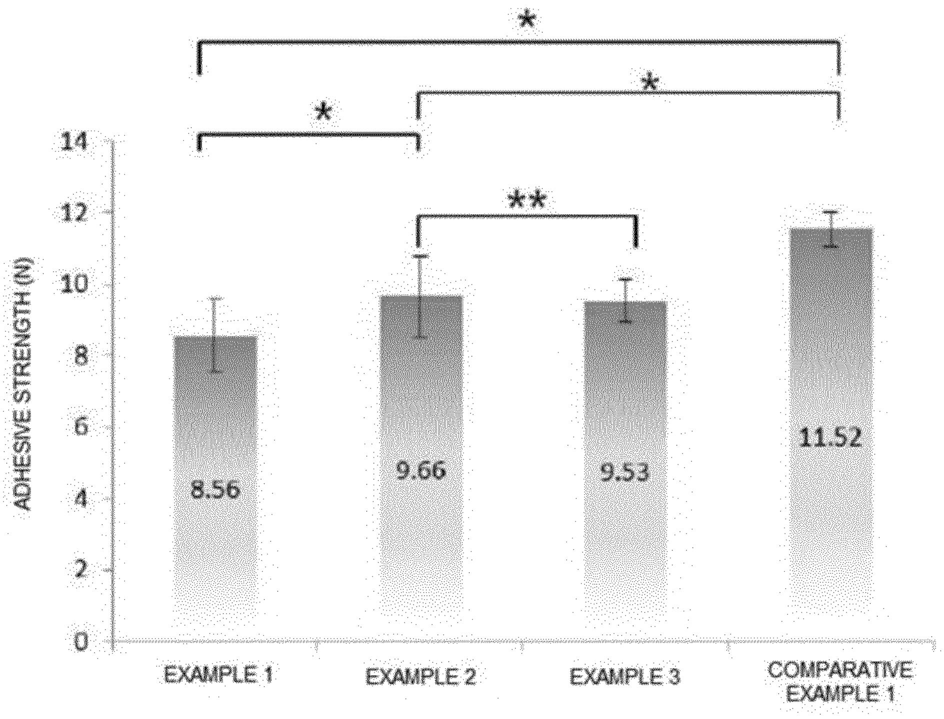
*
*
*
**
14
12
10
8
6
4
2
0
ADHESIVE STRENGTH (N)
8.56
9.66
9.53
11.52
EXAMPLE 1
EXAMPLE 2
EXAMPLE 3
COMPARATIVE EXAMPLE 1

FIG. 5A

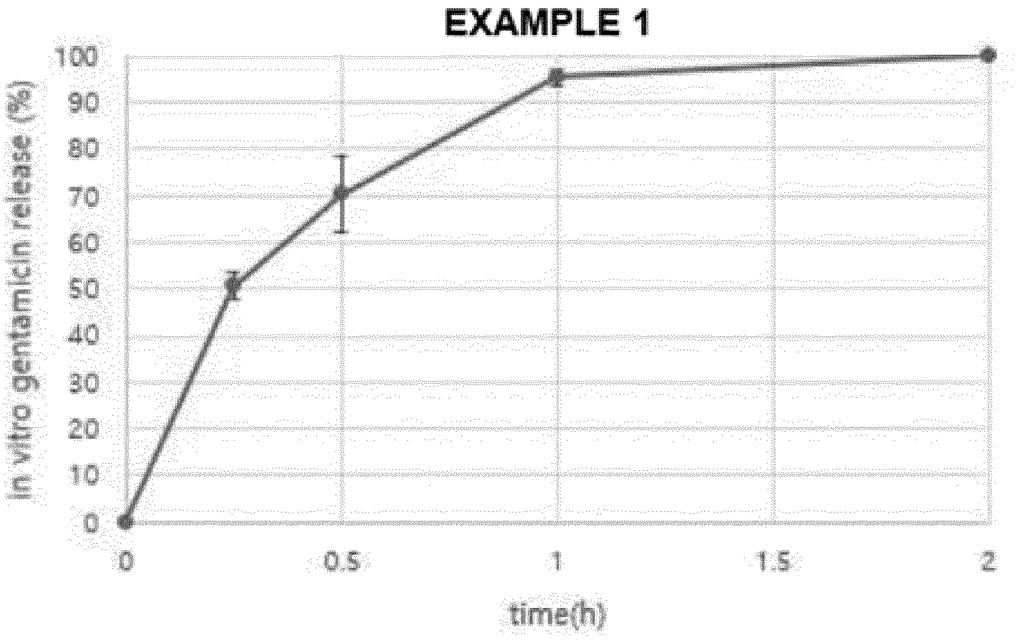
EXAMPLE 1
in vitro gentamicin release (%)
100
90
80
70
60
50
40
30
20
10
0
0
0.5
1
1.5
2
time(h)

FIG. 5B

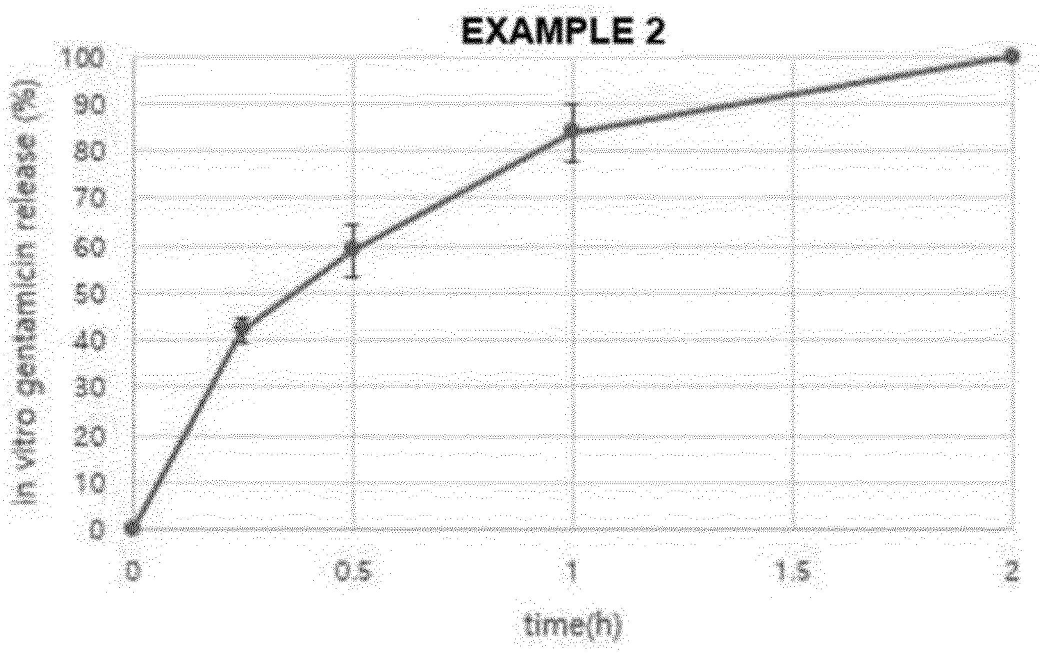
EXAMPLE 2
in vitro gentamicin release (%)
100
90
80
70
60
50
40
30
20
10
0
0
0.5
1
1.5
2
time(h)

FIG. 5C

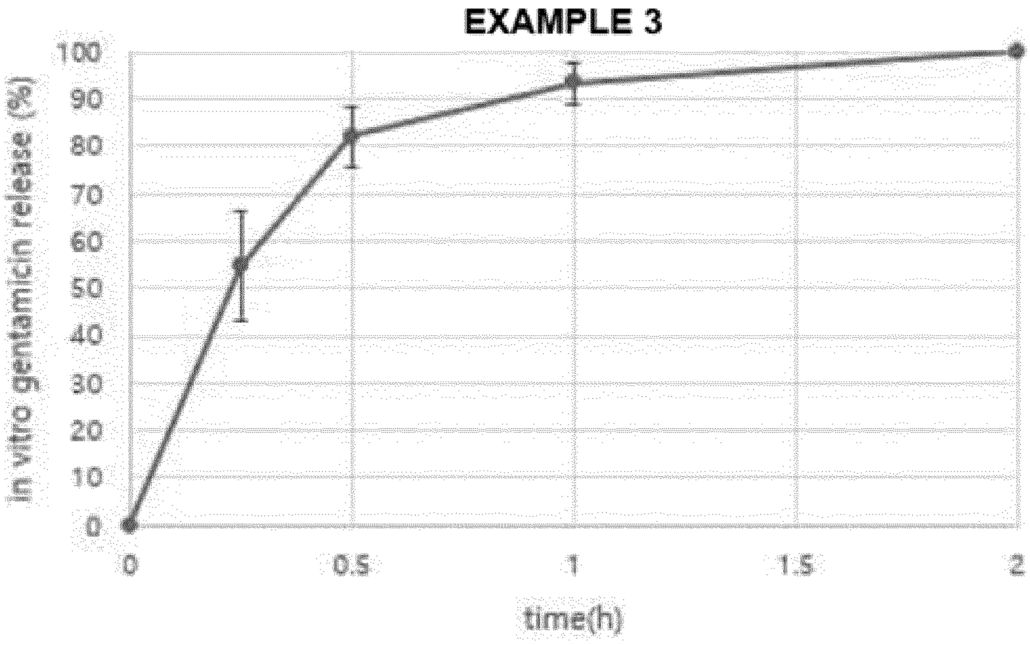
EXAMPLE 3
in vitro gentamicin release (%)
100
90
80
70
60
50
40
30
20
10
0
0
0.5
1
1.5
2
time(h)

FIG. 6

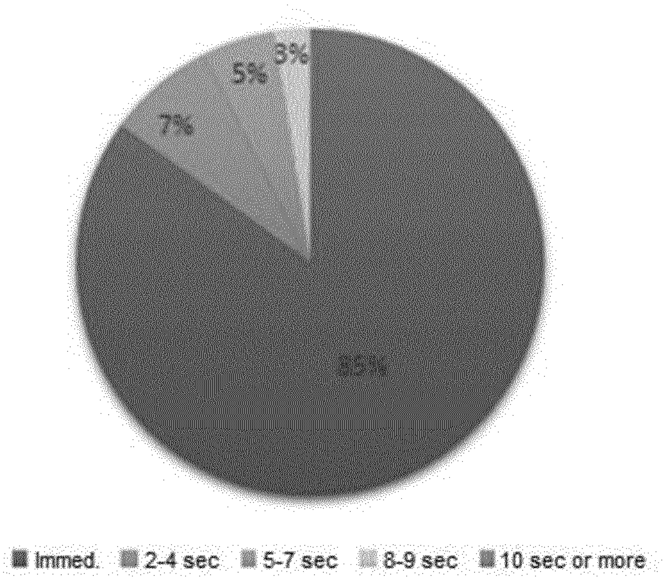
3%
5%
7%
85%
Immed.
2-4 sec
5-7 sec
8-9 sec
10 sec or more

FIG. 7A

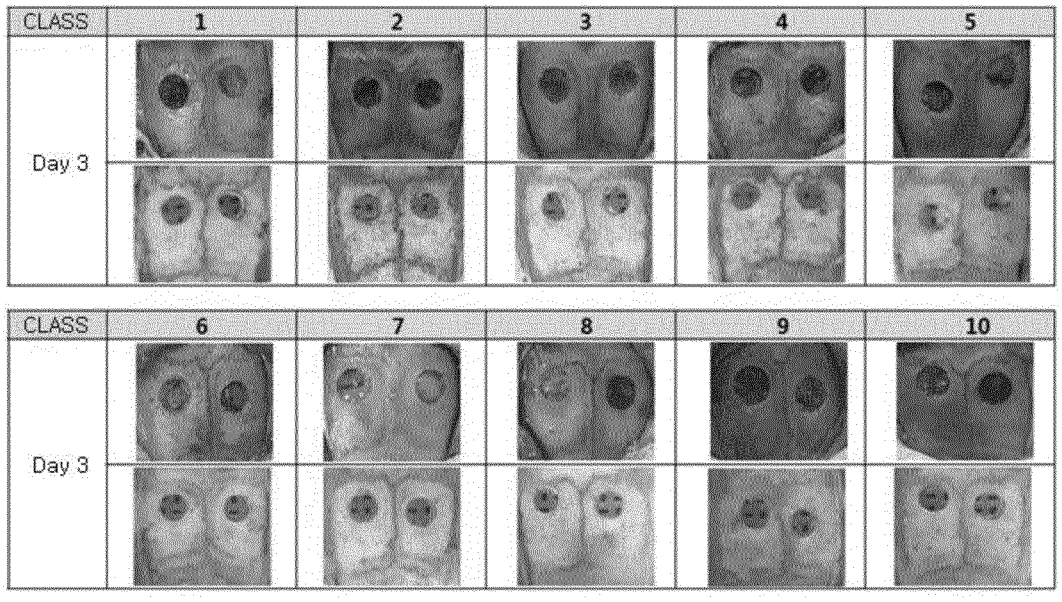
CLASS
1
2
3
4
5
Day 3
CLASS
6
7
8
9
10
Day 3

FIG. 7B

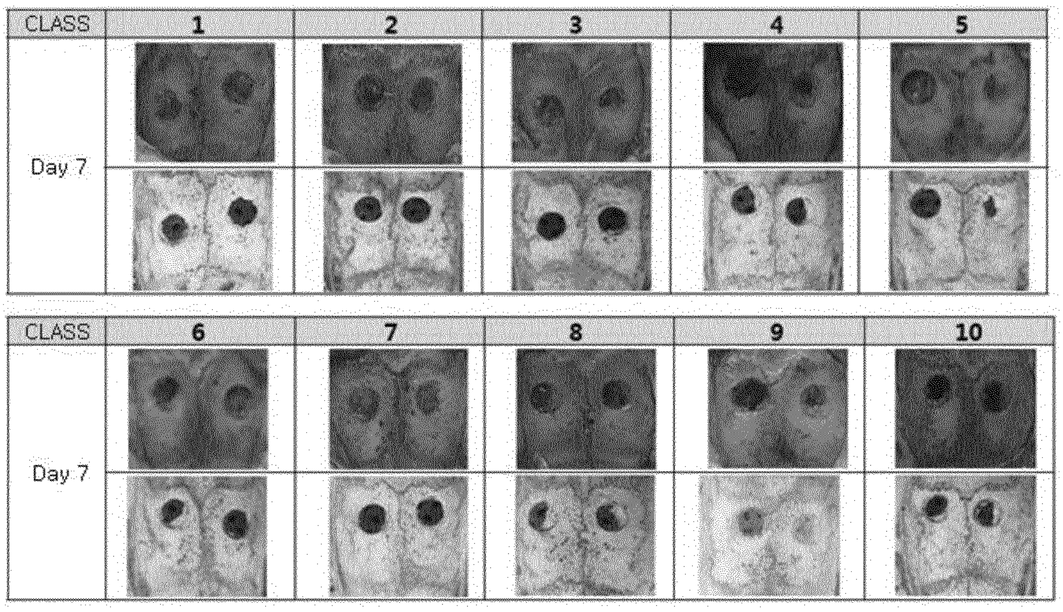
CLASS
1
2
3
4
5
Day 7
CLASS
6
7
8
9
10
Day 7

FIG. 8A

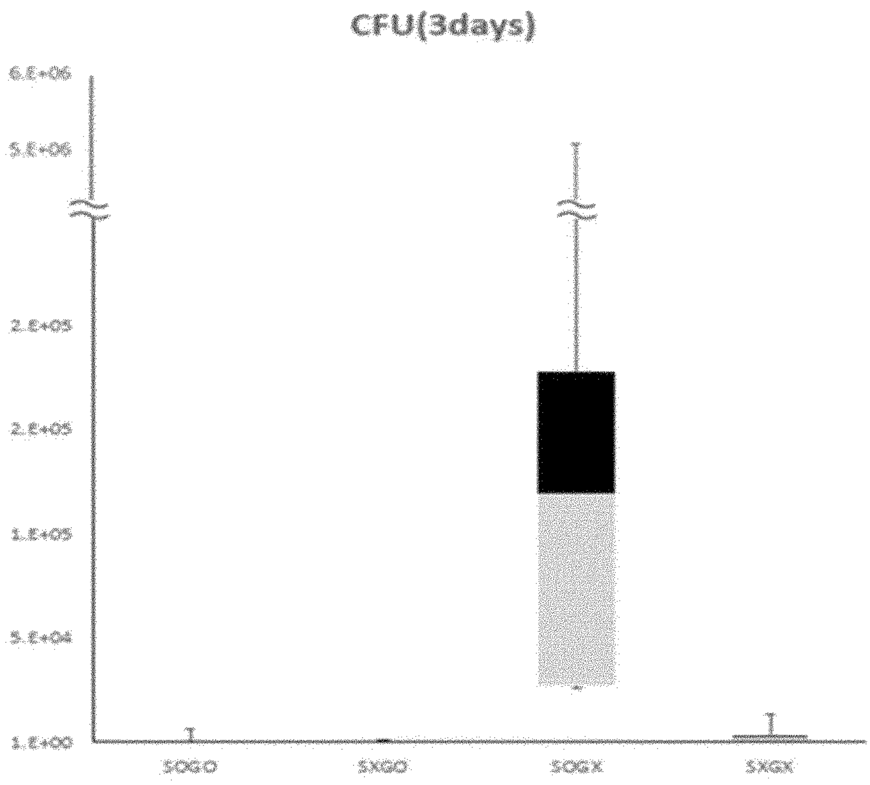
CFU(3days)
6.E+06
5.E+06
2.E+05
2.E+05
1.E+05
5.E+04
1.E+00
SOGO
SXGO
SOGX
SXGX

FIG. 8B

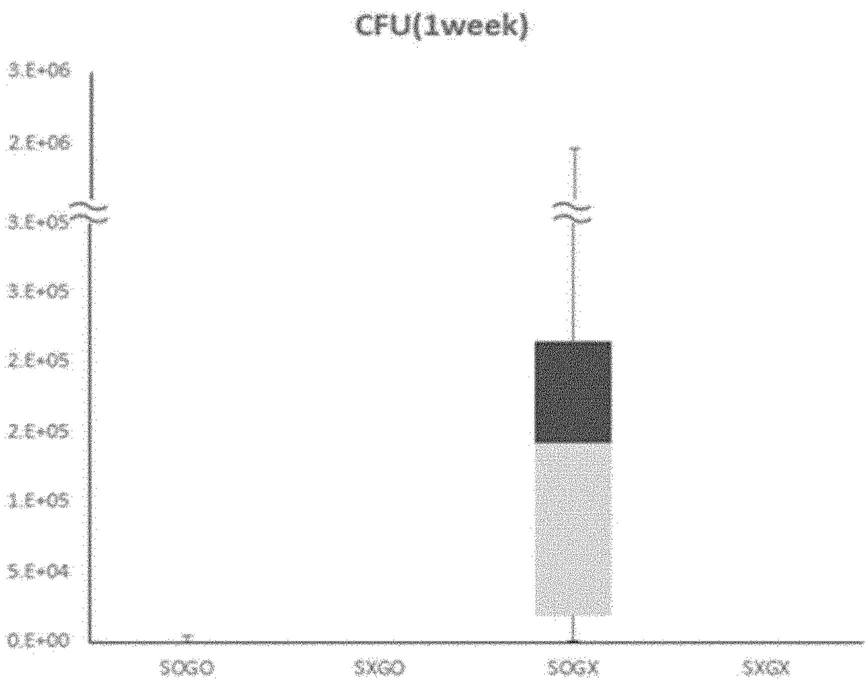
CFU(1week)
3.E+06
2.E+06
3.E+05
3.E+05
2.E+05
2.E+05
1.E+05
5.E+04
0.E+00
SOGO
SXGO
SOGX
SXGX

FIG. 8C

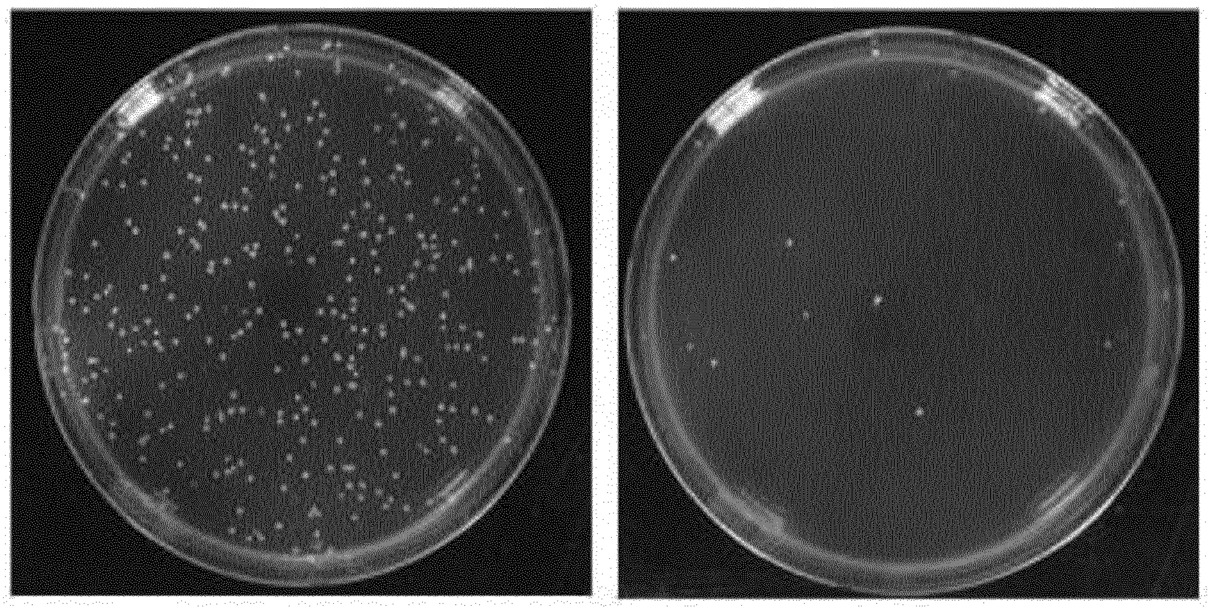

FIG. 8D

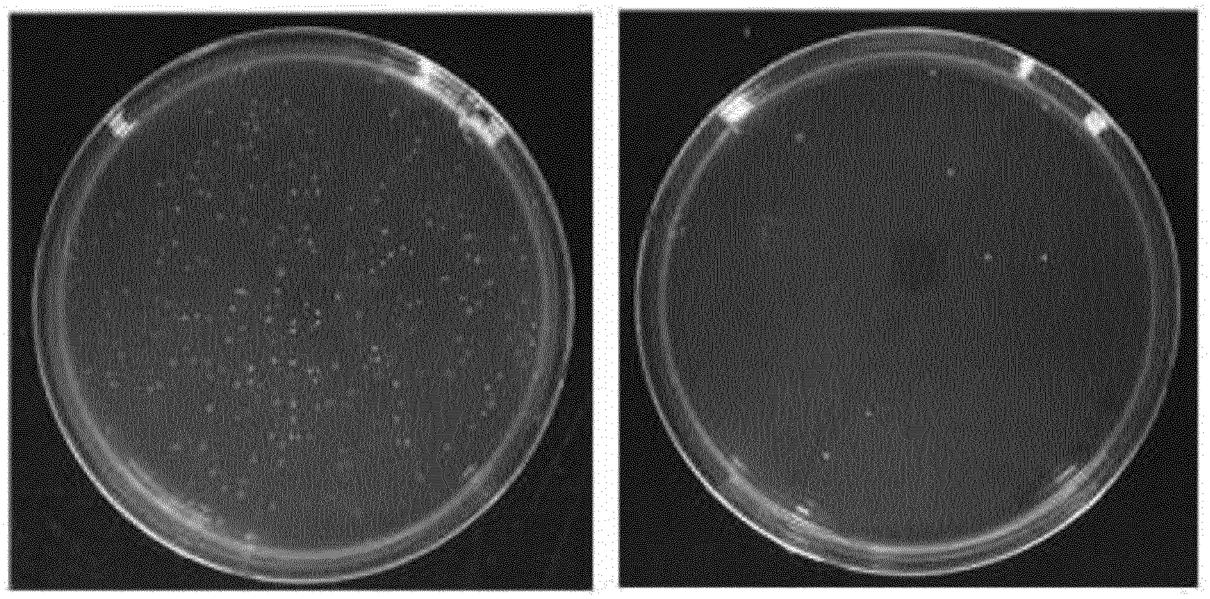

FIG. 9A

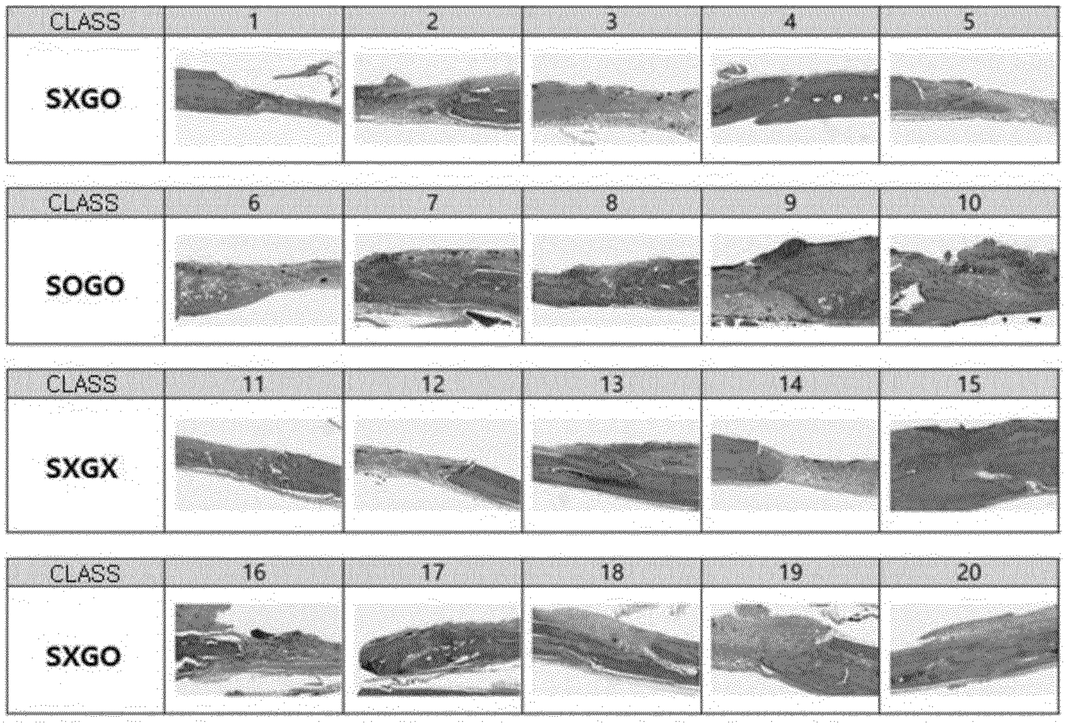
CLASS
1
2
3
4
5
SXGO
CLASS
6
7
8
9
10
SOGO
CLASS
11
12
13
14
15
SXGX
CLASS
16
17
18
19
20
SXGO

FIG. 9B

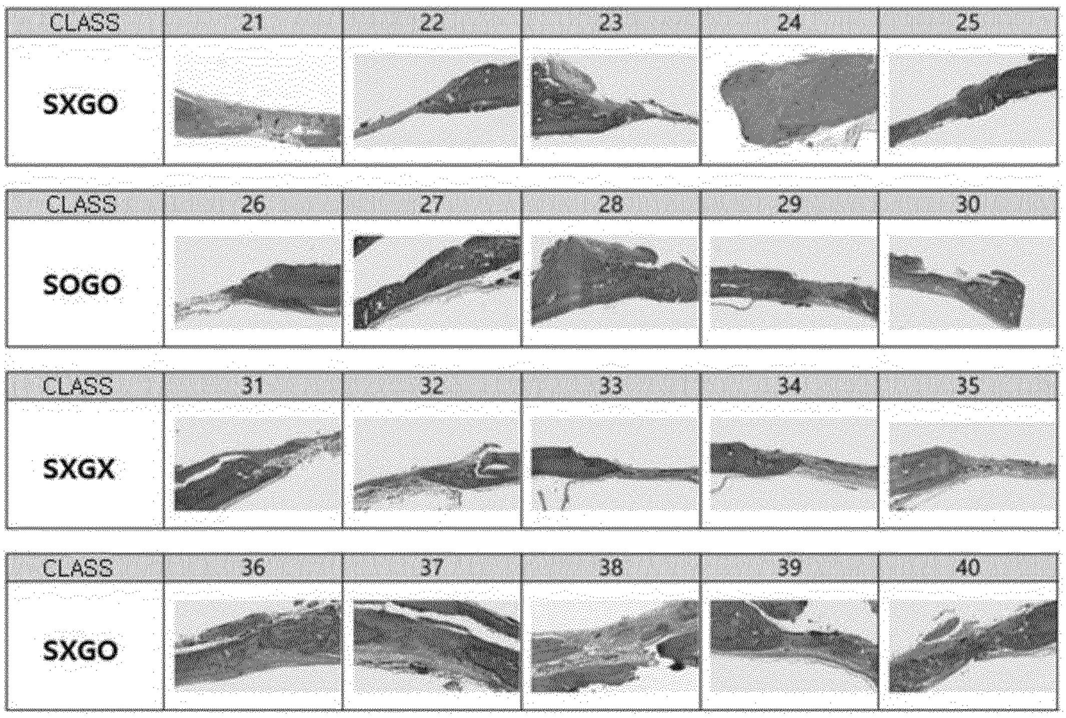
CLASS
21
22
23
24
25
SXGO
CLASS
26
27
28
29
30
SOGO
CLASS
31
32
33
34
35
SXGX
CLASS
36
37
38
39
40
SXGO

FIG. 9C

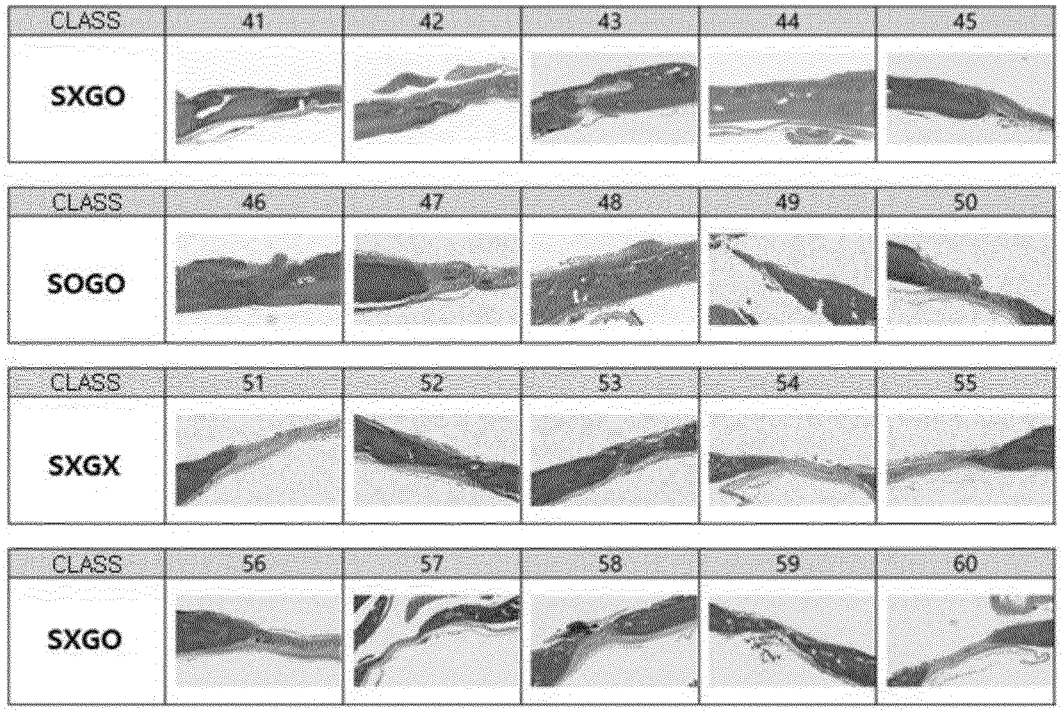
CLASS
41
42
43
44
45
SXGO
CLASS
46
47
48
49
50
SOGO
CLASS
51
52
53
54
55
SXGX
CLASS
56
57
58
59
60
SXGO

FIG. 9D

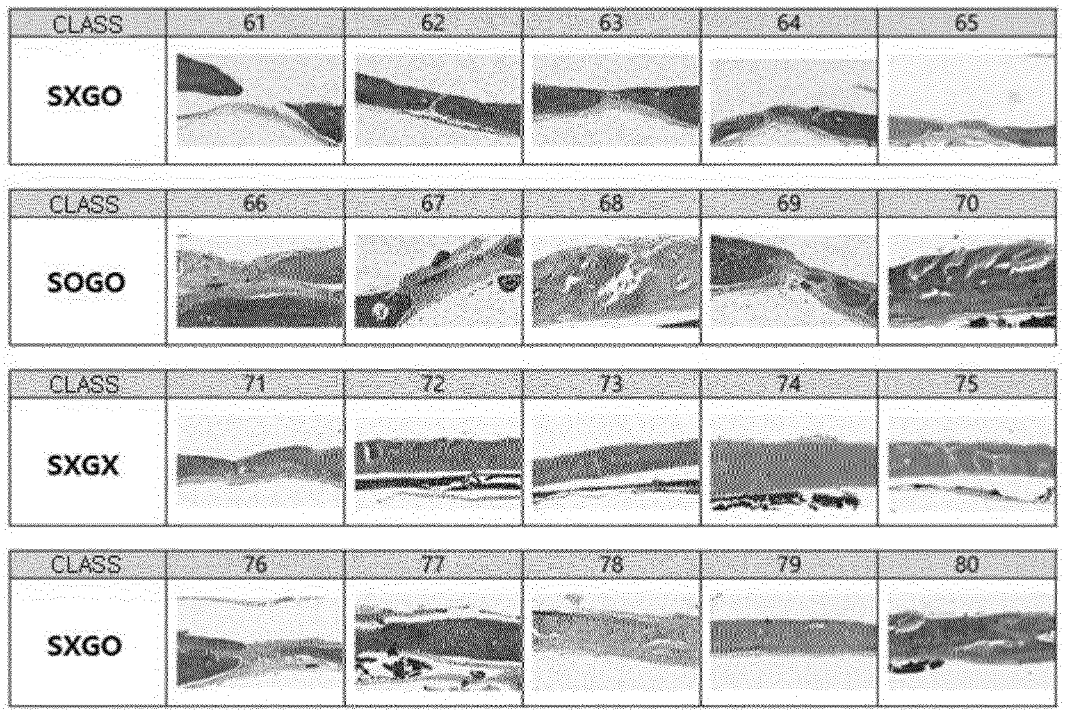
CLASS
61
62
63
64
65
SXGO
CLASS
66
67
68
69
70
SOGO
CLASS
71
72
73
74
75
SXGX
CLASS
76
77
78
79
80
SXGO

**INTERNATIONAL SEARCH REPORT**

International application No.
**PCT/KR2021/013596**

**A. CLASSIFICATION OF SUBJECT MATTER**

**A61L 24/00**(2006.01)i; **A61L 24/04**(2006.01)i; **C08L 71/02**(2006.01)i; **C08K 5/46**(2006.01)i

According to International Patent Classification (IPC) or to both national classification and IPC

**B. FIELDS SEARCHED**

Minimum documentation searched (classification system followed by classification symbols)

A61L 24/00(2006.01); A61K 31/43(2006.01); A61K 31/765(2006.01); A61K 47/34(2006.01); A61K 9/00(2006.01); A61L 15/32(2006.01); A61L 15/44(2006.01); A61L 24/04(2006.01); A61L 27/26(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 흡수성 골 지혈제(absorbent bone hemostat), 폴록사머(poloxamer), 폴리에틸렌 글리콜-란-프로필렌 글리콜(polyethylene glycol-ran-propylene glycol), 항생제(antibiotic), 가열(heating), 교반(stirring), 냉각(cooling)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2020-0088722 A (THERACION BIOMEDICAL CO., LTD.) 23 July 2020 (2020-07-23)<br>See paragraphs [0030]-[0097]; and figures 1-8. | 1-13 |
| Y | KR 10-1990069 B1 (SOLSION BIOMEDICAL INC.) 18 June 2019 (2019-06-18)<br>See paragraphs [0025]-[0071]; and figures 1-7. | 1-13 |
| A | KR 10-1712809 B1 (M.I TECH CO., LTD.) 08 March 2017 (2017-03-08)<br>See entire document. | 1-13 |
| A | JP 2018-027342 A (TRUSTEES OF TUFTS COLLEGE) 22 February 2018 (2018-02-22)<br>See entire document. | 1-13 |
| A | US 2013-0017227 A1 (LAMBERT, C. J., Jr. et al.) 17 January 2013 (2013-01-17)<br>See entire document. | 1-13 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2022** | **14 March 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/013596**

| Patent document cited in search report | Publication date (day/month/year) | Patent family member(s) | Publication date (day/month/year) |
|---|---|---|---|
| KR 10-2020-0088722 A | 23 July 2020 | EP 3912651 A1 | 24 November 2021 |
| | | KR 10-2211765 B1 | 03 February 2021 |
| | | WO 2020-149449 A1 | 23 July 2020 |
| KR 10-1990069 B1 | 18 June 2019 | KR 10-2019-0061778 A | 05 June 2019 |
| | | WO 2019-107588 A1 | 06 June 2019 |
| KR 10-1712809 B1 | 08 March 2017 | KR 10-2016-0112511 A | 28 September 2016 |
| JP 2018-027342 A | 22 February 2018 | AU 2010-257120 A1 | 09 December 2010 |
| | | AU 2015-201538 A1 | 16 April 2015 |
| | | AU 2017-200570 A1 | 23 February 2017 |
| | | AU 2018-267539 A1 | 13 December 2018 |
| | | CA 2791580 A1 | 09 December 2010 |
| | | JP 2012-519698 A | 30 August 2012 |
| | | JP 2016-104724 A | 09 June 2016 |
| | | JP 2020-058834 A | 16 April 2020 |
| | | JP 5909362 B2 | 26 April 2016 |
| | | JP 6301896 B2 | 28 March 2018 |
| | | US 2012-0052124 A1 | 01 March 2012 |
| | | US 2014-0105995 A1 | 17 April 2014 |
| | | US 2019-0175785 A1 | 13 June 2019 |
| | | WO 2010-141133 A2 | 09 December 2010 |
| | | WO 2010-141133 A3 | 31 March 2011 |
| US 2013-0017227 A1 | 17 January 2013 | US 2013-0230566 A1 | 05 September 2013 |
| | | WO 2013-012725 A1 | 24 January 2013 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020140107429
- KR 1020180055747